# EUROPEAN PATENT SPECIFICATION

(11) **EP 2 051 770 B1**
(45) Date of publication and mention of the grant of the patent: **19.06.2013**
(21) Application number: 07766306.0
(22) Date of filing: 19.07.2007
(51) Int. Cl.: A61M 39/10

(54) **FLUID CONNECTOR SYSTEM**
FLUID-VERBINDUNGSSYSTEM
SYSTÈME DE CONNECTEURS POUR FLUIDE

(30) Priority: 20.07.2006 GB 0614452
(43) Date of publication of application: 29.04.2009
(73) Proprietor: Young, Peter Jeffrey, Norfolk PE32 1AR (GB)
(72) Inventor: Young, Peter Jeffrey, Norfolk PE32 1AR (GB)
(74) Representative: Gibson, Mark
(86) International application number: PCT/GB2007/002741
(87) International publication number: WO 2008/009946

(56) References cited:
- WO-A-2005/055919
- GB-A- 2 383 828
- US-A- 5 286 067
- US-B1- 6 612 624

## Description

The invention provides a connector system for connecting conduits for fluid flow therebetween.

In hospitals and medical facilities, the standard method for attaching infusion equipment such as syringes and pumps to catheters and IV tubes and other similar equipment *in situ* in a patient involves the use of the Luer connector system. Luer connectors consist of male and female connector parts which mutually interengage in a fluid-tight manner, either by a simple interference fit, known as a slip or, in a variation, by a simple thread, known as a lock.

In the system which is currently in use around the world, the convention that has been adopted is for the connector part at the patient-end, i.e. on the IV tube or catheter *in situ* in the patient to be the female connector part. This is because, in general, syringes are made with male Luer connector parts. Clearly, standardisation of connectors is an advantage of the system as it avoids equipment incompatibility problems in large organisations such as hospitals which have complicated and diverse equipment requirements.

A problem which has arisen with the Luer connection system is unfortunately also a result of this standardisation.

It is sometimes desirable to supply fluid in the medical environment that is suitable for infusion to one space but not another. For example, a connector from a local anaesthetic infusion or syringe may be required to attach to a connector on an epidural catheter but should not be connected to an intravenous catheter or an intrathecal catheter. Another example is a drug infusion that is suitable for central venous infusion that would be dangerous given through a peripheral venous cannula.

Scott describes a system of non-interchangeable connectors that are uniquely configured male/female pairs whereby only complementary pairs of a set will fit together. Scott's system demands that only infusions or injections of the proper type be connected to one another.

The fact that the Scott system relies on having connectors which will only connect with each other gives rise to a number of problems. Thus, in an emergency, the correct complementary Scott connector may not be immediately available. Having said that, however, standard male slip and lock Luer connectors are ubiquitous in hospital and clinic environments and it is desirable that clinicians should be able to give infusions or injections using these connectors.

It is an object of the invention to seek to mitigate these problems.

US 5,286,067 describes a shrouded tubing interface for quickly connecting, disconnecting and avoiding contamination of first and second fluid flow lines comprising male and female connectors. It discloses the combination of features of the preamble of claim 1.

The invention is defined in claim 1. There is provided a connector system for connecting conduits for fluid flow therebetweeen, the system comprising a first male connector and a first female connector, the connectors being releasably interengageable to form a fluid-tight connection, the first male connector being adapted to connect to the first female connector, but not to a standard female locking Luer connector, and the first female connector being adapted to connect to the first male connector and a standard male slip and/or locking Luer connector.

Accordingly, the invention makes the novel female connector of the invention compatible with the standard Luer system so that standard male Luer slip and/or lock connectors will fit onto the novel female connector of the invention. However, the novel male connector of the invention will not fit onto a standard female lock Luer connector. Thus, clinicians may use standard male slip and/or lock Luer connectors to give infusions or injections. This is important as it is more convenient for a hospital to introduce new infusion equipment in stages and this demands compatibility of the current standard infusion equipment with the novel female connectors on the infusion devices inserted in the patient. Also, a manufacturer might not want to make a catheter with a connector that is not compatible with current standard Luer locking equipment as this would limit distribution.

The first male connector may comprise a screw thread and means for preventing the screw thread of a standard female locking Luer connector from engaging with said screw thread.

The first female connector may comprise a screw thread and the first male connector may comprise means for allowing said screw thread to engage with the screw thread of the first male connector.

In a first arrangement, the means for preventing the screw thread of a standard female locking Luer connector from engaging with the screw thread of the first male connector comprises a barrier.

The first male connector may comprise a first conduit held within a sleeve, the screw thread being positioned on the inside of the sleeve, the barrier being connected to or part of the sleeve, and being positioned at the entrance or within the sleeve.

The barrier may be such that at least part of the sleeve has an internal cross-section small enough to prevent a standard female locking Luer connector from engaging with the screw thread of the first male connector.

The barrier may comprise a ridge around all or part of the inner circumference of the sleeve.

The barrier may be part of the sleeve, and the shape of the sleeve may be such that a standard female locking Luer connector is prevented from engaging with the screw thread of the first male connector.

The means for allowing the screw thread of the first female connector to engage with the screw thread of the first male connector may comprise aperture(s) in the barrier.

The first female connector may comprise a second conduit and the screw thread may comprise one or more projection(s) on the outside of said second conduit, the first conduit being insertable in the second conduit to form a connection between the two conduits, the aperture(s) in the barrier on the first male connector being positioned so as to allow passage to the projection(s) on the first female connector.

The system may comprise a plurality of male and female connectors, and the barrier on the first male connector and the projection(s) on the first female connector may be such that they prevent all the other female connectors from engaging with the screw thread of the first male connector.

Alternatively, the system may comprise a plurality of male and female connectors, and the barrier on the first male connector and the projection(s) on the first female connector may be such that they allow at least one of the other female connectors to engage with the screw thread of the first male connector.

Each male connector may comprise means to guide the each female connector on insertion of the first conduit into the second conduit.

The guide means may be tapered towards the entrance of the sleeve.

The guide means may comprise the barrier.

Each male connector may comprise orientation means to assist with positioning each female connector so that the projection(s) are in the correct orientation to pass through the aperture(s) on insertion the first conduit into the second conduit.

The orientation means may comprise markers at the entrance to the sleeve of each male connector.

The diameter of the second conduit of the first female connector may be substantially the same as that of the second conduit in a standard female slip Luer connector. The first female connector will then be able to connect with a standard male slip Luer connector.

The diameter of the first female connector at the point where the projection(s) are attached to the connector may be substantially the same as that of a standard female locking Luer connector. The first female connector will then also be able to connect with a standard male locking Luer connector.

In a second arrangement, the means for preventing the screw thread of a standard female locking Luer connector from engaging with the screw thread of the first male connector comprises providing the first male connector with a screw thread which is not engageable with the screw thread of a standard female locking Luer connector.

The means for allowing the screw thread of the first female connector to engage with the screw thread of the first male connector may comprise providing the first female connector with a screw thread which is engageable with the screw thread of the first male connector.

The first female connector may comprise a screw thread which is engageable with the screw thread of a standard male locking Luer connector.

The first male connector may comprise a plurality of screw threads, each screw thread of the first male connector being engageable with the screw thread of a different female connector.

The first female connector may comprise a plurality of screw threads, each screw thread of the first female connector being engageable with the screw thread of a different male connector.

According to a second aspect of the invention, there is provided epidural, intrathecal or intravenous apparatus, including a system as defined above.

The invention will now be illustrated by way of example with reference to the following drawings, of which:
Figures 1 and 2 show a prior art standard female locking Luer connector;
Figures 3 and 4 show a prior art standard male locking Luer connector;
Figure 5 shows the prior art female and male connectors linked;
Figure 6 shows a first embodiment of a connector system according to the invention;
Figure 7 shows a second embodiment of a connector system according to the invention;
Figure 8 shows a third embodiment of a connector system according to the invention;
Figure 9 shows a first modification of the male connectors shown in Figures 6 to 8;
Figure 10 shows a second modification of the male connectors shown in Figures 6 to 8;
Figure 11 shows a third modification of the male connectors shown in Figures 6 to 8;
Figure 12 shows a fourth modification of the male connectors shown in Figures 6 to 8; and
Figure 13 shows a fifth modification of the male connectors shown in Figures 6 to 8;

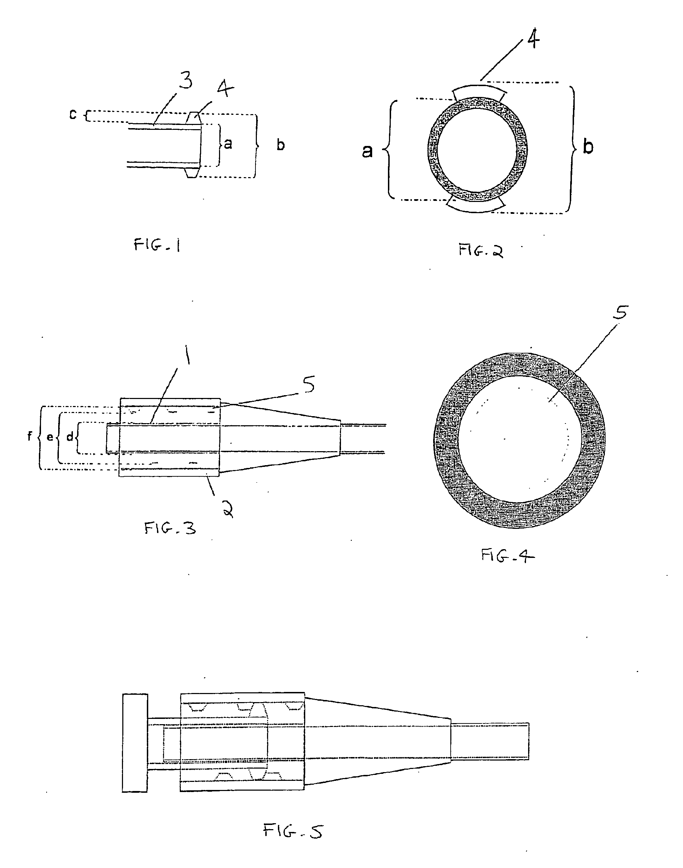

In prior art, commonly the connector at the patient side of an infusion connection is a female Luer lock connector (Figures 1 and 2), and commonly the connector at the syringe or pump side of an infusion connection is a male Luer lock connector (Figures 3 and 4).

The male Luer connector has a first conduit (1) held within a sleeve (2). The female Luer connector has a second conduit (3). The outside of the second conduit (3) carries a number of protuberances (4). The inside of the sleeve (2) carries a screw thread (5). In use, the first conduit (1) is inserted in the second conduit (3) to form a fluid-tight connection between the two connectors, and the protuberances (4) of the standard female Luer connector engage the screw thread (5) of the standard male Luer connector (2) as shown in Figure 5.

The dimensions a-f of current standard Luer connectors are indicated in European Standard EN1707 of January 1997.

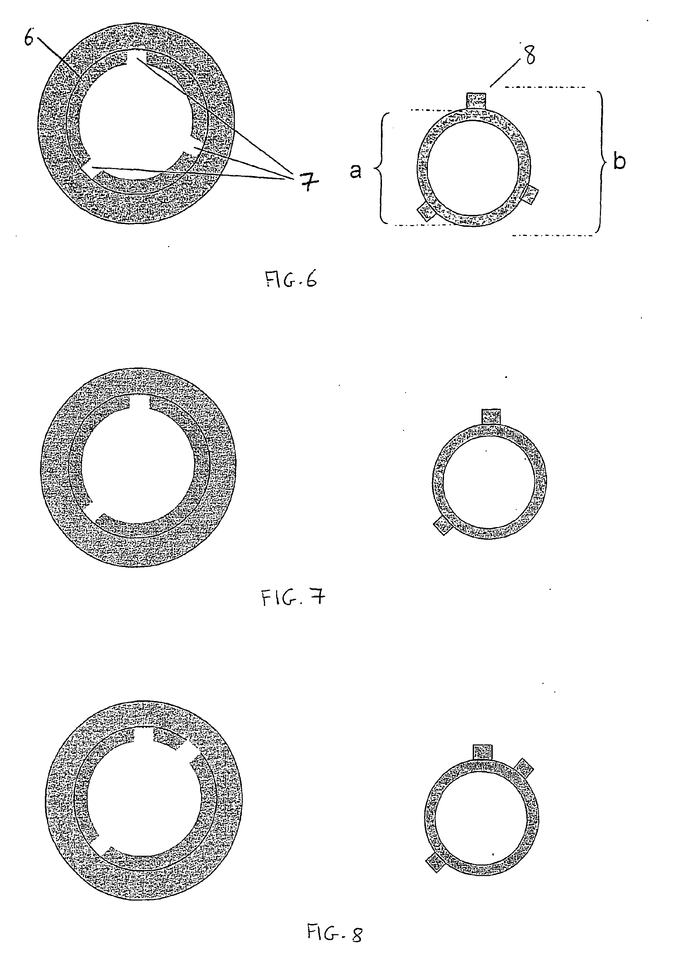

Figures 6 to 8 show three embodiments of the present invention.

Each embodiment comprises a male Luer connector with an additional disc (6) which has a notch or series of notches (7) in the circumference. The inner diameter of each disc is close to the inner diameter of the standard Luer thread (b). For current Luer connectors, the disc (6) requires an internal diameter of 6.5 mm to 7mm.

The notches (7) in the disc (6) in each of the male connectors are positioned such that they will prevent the passage of the protuberances (4) on a standard female connector and, therefore, the male Luer connector is prevented from connecting with a standard locking Luer female connector. It is possible for each male connector to connect to a standard slip Luer female connector as such a connector will have no protuberances. However, this is of little consequence as current female Luer intravenous and epidural connectors normally have protuberances and so will not connect to the male connectors.

Each embodiment also comprises a female Luer connector with protuberances (8) that are smaller and/or on a different circumferential position of the disc to the protuberances (1) of the standard female locking Luer connector, but which will still engage normally the screw thread (5) of the standard male locking Luer connection. It is also possible for each male connector to connect to a standard slip female Luer connector

An advantage of the current invention over simply changing the shape of the Luer connectors to achieve unique connectability is that there is a continuation of compatibility of current standard syringe and infusion slip connectors to the novel female connector. The clinician, therefore, has a choice to use a syringe or infusion tubing with a standard prior art male connector if a syringe or infusion tubing for the current novel male connector were not available. This would make the introduction of these novel connectors easier in a hospital as, if the novel male connector were not available in a clinical environment, then that patient could be provided with an injection or infusion through a standard male slip Luer connector.

The relative positions of the notches (7) and protuberances (8) on the male and female connectors will determine the connectivity of the connectors.

For example, the male connector shown in Figure 6 will connect with the female connectors shown in Figures 6 and 7, but not the female connector shown in Figure 8, whereas the male connector shown in Figure 7 will connect only with the female connector shown in Figure 7.

It may be desirable for all connectors within an infusion system to be of the same key, that is, the notches (7) in the disc (6) would be the same at all points in line with matching female connectors to ensure the correct infusion is given to the correct patient line. That is, infusion syringe to line and line to patient.

One embodiment would have only a male epidural connector fitting with a female epidural catheter connector, only a male intrathecal connector fitting with a female intrathecal catheter connector and only a male intravenous connector fitting with a female intravenous catheter connector. However, in each case, the female connectors will still fit with a standard male locking Luer connector or a standard slip end syringe. This is because the dimensions a, b, c and d remain unchanged from the standard Luer dimensions.

A suitable configuration for an epidural catheter and infusion set would be as shown in Figure 8. However, there are clearly many other possible configurations.

Some drug infusions are suitable to be given through a central venous line but not through a peripheral line. It is desirable for a central venous line to have a different key to peripheral lines. Drugs suitable for peripheral infusion, however, are normally suitable for peripheral infusion. A connector for the female connector of the central venous line, therefore, should accept both the male connector for peripheral infusions and the male connector for central venous lines. The peripheral infusion female connector, however, should accept only the male peripheral connector and not the male central venous connector.

One embodiment to achieve this would be having peripheral infusion connectors such as those shown in Figure 6, and central venous connectors such as those shown in Figure 7. However, there are clearly many other possible configurations.

It would normally be desirable for all components of the infusion system to have the same connector key to ensure misconnection does not occur at any point in the infusion system. For example in the embodiment of the invention having a peripheral venous cannula with a female connector as in Figure 6 and a central venous catheter with a female connector as in Figure 7, the infusion tubing and the syringe connector and all taps within the infusion line for medicines suitable for central venous administration only should have male and female connectors as shown in Figure 7.

Connectors to arterial lines should accept only arterial line tubing and female side port connectors should not accept any Luer connectors - that is the protuberances (8) should be incompatible with all other key notches.

The male and female connectors may have markings as an indication of orientation to assist the clinician with lining up the notches (7) in the male connector with the protuberances (8) in the female connector.

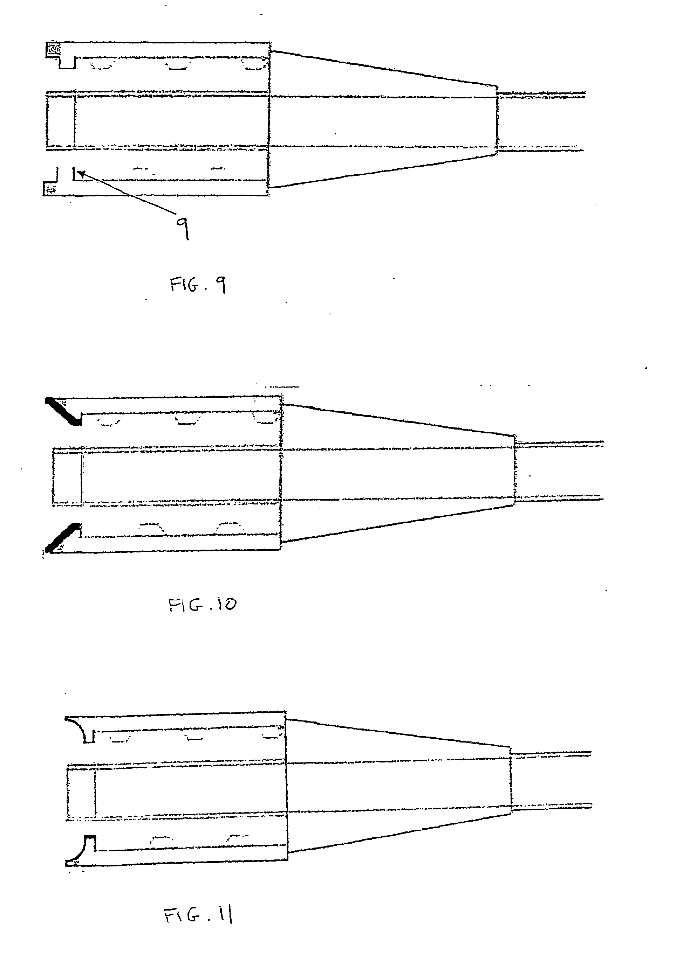

The male connector may have a ridge modification or indentation modification (9) at the entrance to the connector. The ridge or indentation helps to guide the female component into place into a standard luer-lock or a modified luer-lock. This can have perpendicular straight (Figures 9 and 10) or curved edges (Figure 11) or edges with other surface configurations.

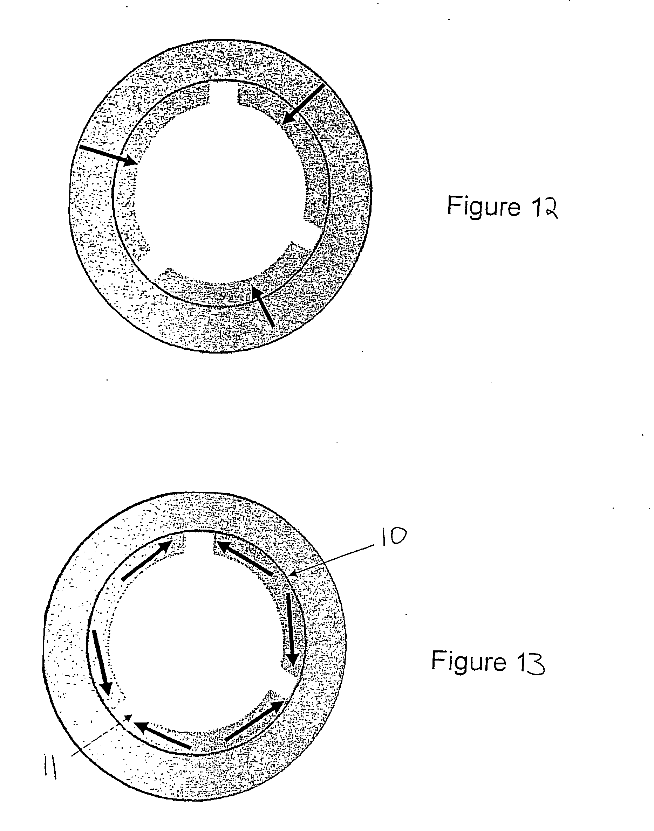

When attempting to fit together a Luer connection or modified Luer connection, it can be difficult to attach the male and female components if they are not correctly aligned. Another embodiment of the rim modification comprises radial (Figure 12) or circumferential (Figure 13) slopes as shown by the arrows. This sloping helps to improve alignment. The circumferential slopes cause a wave profile with nadirs at the gaps (11) and peaks at the midpoints (10). This helps to guide the protrusions on the female connector into the male Luer-lock or slip connector.

Another embodiment of the rim modification comprises a combination of both radial and circumferential slopes that can improve alignment.

Another embodiment of the rim modification comprises a radial and/or circumferential slope on the inner surface (Figure 12). This helps disengagement of the attached connector.

Any individual, combination or all of these embodiments can be used together to improve the alignment of the connector.

Needles or devices to draw up drugs may carry a coding system. The drawing up ports may be integral to the drug container or else the device may be colour coded to match with the colour codes on the drug ampoule to indicate compatibility with the infusion system and, therefore, the intended site of infusion.

The connectors, syringes, needles and the like may be colour coded or otherwise identified to assist the identification of the connector type and the suitability for correct usage. It would also be advantageous for the drug ampoules to be identified by colour coding, or otherwise, as to suitable infusion route.

The spike at the proximal end of an infusion set for connection to a local anaesthetic solution in a bag is currently also of a standardised size. Reducing the spike size of the proximal end of the current invention infusion achieves the ability to produce local anaesthetic bags with a smaller apperture than the current prior art. This is advantageous because novel connector local anaesthetic infusion bags can then only be connected to novel local anaesthetic administration systems. The complication of accidental connection to intravenous systems is thus avoided. The smaller diemeter spike retains backward compatability with current local anaesthetic bags. To achieve a seal with prior art bags a variable (tapering or stepwise) spike diameter may be desirable.

In each of the embodiments described above, the screw thread of a standard female locking Luer connector is prevented from engaging with the screw thread of the male connector by means of a barrier (disc (6)). However, any suitable obstruction may be used.

For example, in an alternative arrangement (not illustrated), the male connector is provided with a screw thread ("screw thread A") which is not engageable with the screw thread of a standard female locking Luer connector ("screw thread B"). The female connector is also provided with screw thread A so that the female connector will be engageable with the male connector. The female connector may optionally also be provided with screw thread B so that it will be engageable with a standard male locking Luer connector.

Multiple screw threads may be used in both the male and female connectors where multiple unique systems are required.

## Claims

1. A connector system for connecting conduits for fluid flow therebetweeen, the system comprising:
a) a first male connector; and
b) a first female connector,
wherein the connectors are releasably interengageable to form a fluid-tight connection,
wherein the first male connector is adapted to connect to the first female connector, but not to a standard female locking Luer connector by means of a screw thread which is not engageable with the screw thread (4) of the standard female locking Luer connector,
**Characterised in that** the first female connector is adapted to connect to the first male connector and a standard male locking Luer connector by means of a screw thread which is engageable with the screw thread of the first male connector and a screw thread (5) of a standard male locking Luer connector, and
wherein the first male connector comprises a plurality of screw threads, each screw thread of the first male connector being engageable with the screw thread of a different female connector.

2. A connector system according to claim 1, wherein the first male connector comprises a screw thread and means for preventing the screw thread (4) of a standard female locking Luer connector from engaging with said screw thread.

3. A connector system according to claim 2, wherein the first female connector comprises a screw thread and the first male connector comprises means for allowing said screw thread to engage with the screw thread of the first male connector.

4. A connector system according to claim 2 or claim 3, wherein the means for preventing the screw thread (4) of a standard female locking Luer connector from engaging with the screw thread of the first male connector comprises a barrier (6).

5. A connector system according to claim 4, wherein the first male connector comprises a first conduit (1) held within a sleeve (2), the screw thread being positioned on the inside of the sleeve (2), the barrier (6) being connected to or part of the sleeve (2), and being positioned at the entrance or within the sleeve (2).

6. A connector system according to claim 5, wherein the barrier (6) is such that at least part of the sleeve (2) has an internal cross-section small enough to prevent a standard female locking Luer connector from engaging with the screw thread of the first male connector.

7. A connector system according to any of claims 4 to 6, wherein the means for allowing said screw thread of the first female connector to engage with the screw thread of the first male connector comprises aperture(s) (7) in the barrier (6).

8. A connector system according to claim 7, wherein the first female connector comprises a second conduit (3) and the screw thread comprises one or more projection(s) (8) on the outside of said second conduit (3), the first conduit (1) being insertable in the second conduit (3) to form a connection between the two conduits, the aperture(s) (7) in the barrier (6) on the first male connector being positioned so as to allow passage to the projection(s) (8) on the first female connector.

9. A connector system according to claim 8, wherein the system comprises a plurality of male and female connectors, and the barrier (6) on the first male connector and the projection(s) (8) on the first female connector are such that they prevent all the other female connectors from engaging with the screw thread of the first male connector.

10. A connector system according to claim 8, wherein the system comprises a plurality of male and female connectors, and the barrier (6) on the first male connector and the projection(s) (8) on the first female connector are such that they allow at least one of the other female connectors to engage with the screw thread of the first male connector.

11. Epidural, intrathecal, regional anaesthesia, local anaesthesia or intravenous apparatus, including a system according to any of claims 1 to 10.

## Patentansprüche

1. Verbindersystem zum Verbinden von Leitungen für einen Fluidfluss dazwischen, wobei das System Folgendes umfasst:
a) ein erstes männliches Verbindungsstück; und
b) ein erstes weibliches Verbindungsstück,
wobei die Verbindungsstücke lösbar miteinander in Eingriff gebracht werden können, um eine fluiddichte Verbindung herzustellen,
wobei das erste männliche Verbindungsstück zum Verbinden mit dem ersten weiblichen Verbindungsstück, aber nicht mit einem standardmäßigen weiblichen Luer-Verriegelungsverbinder mittels eines Schraubgewindes ausgelegt ist, das nicht mit dem Schraubgewinde (4) des standardmäßigen weiblichen Luer-Verriegelungsverbinders in Eingriff gebracht werden kann,
**dadurch gekennzeichnet, dass** das erste weibliche Verbindungsstück zum Verbinden mit dem ersten männlichen Verbindungsstück und einem standardmäßigen männlichen Luer-Verriegelungsverbinder mittels eines Schraubgewindes ausgelegt ist, das mit dem Schraubgewinde des ersten männlichen Verbindungsstücks und einem Schraubgewinde (5) eines standardmäßigen männlichen Luer-Verriegelungsverbinders in Eingriff gebracht werden kann, und
wobei das erste männliche Verbindungsstück mehrere Schraubgewinde aufweist, wobei jedes Schraubgewinde des ersten männlichen Verbindungsstücks mit dem Schraubgewinde eines anderen weiblichen Verbindungsstücks in Eingriff gebracht werden kann.

2. Verbindersystem nach Anspruch 1, wobei das erste männliche Verbindungsstück ein Schraubgewinde sowie Mittel umfasst, um zu verhindern, dass das Schraubgewinde (4) eines standardmäßigen weiblichen Luer-Verriegelungsverbinders mit dem genannten Schraubgewinde in Eingriff kommt.

3. Verbindersystem nach Anspruch 2, wobei das erste weibliche Verbindungsstück ein Schraubgewinde umfasst und das erste männliche Verbindungsstück Mittel umfasst, um es zuzulassen, dass das genannte Schraubgewinde mit dem Schraubgewinde des ersten männlichen Verbindungsstücks in Eingriff kommt.

4. Verbindersystem nach Anspruch 2 oder Anspruch 3, wobei das Mittel zum Verhindern eines Eingriffs zwischen dem Schraubgewinde (4) eines standardmäßigen weiblichen Luer-Verriegelungsverbinders und dem Schraubgewinde des ersten männlichen Verbindungsstücks eine Barriere (6) aufweist.

5. Verbindersystem nach Anspruch 4, wobei das erste männliche Verbindungsstück eine in einer Hülse (2) gehaltene erste Leitung (1) umfasst, wobei das Schraubgewinde auf der Innenseite der Hülse (2) positioniert ist, wobei die Barriere (6) mit der Hülse (2) oder einem Teil davon verbunden und am Eingang oder in der Hülse (2) positioniert ist.

6. Verbindersystem nach Anspruch 5, wobei die Barriere (6) derart ist, dass wenigstens ein Teil der Hülse (2) einen Innenquerschnitt hat, der klein genug ist, um einen Eingriff zwischen einem standardmäßigen weiblichen Luer-Verriegelungsverbinder und dem Schraubgewinde des ersten männlichen Verbindungsstücks zu verhindern.

7. Verbindersystem nach einem der Ansprüche 4 bis 6, wobei das Mittel zum Zulassen eines Eingriffs zwischen dem genannten Schraubgewinde des ersten weiblichen Verbindungsstücks und dem Schraubgewinde des ersten männlichen Verbindungsstücks (eine) Öffnung(en) (7) in der Barriere (6) aufweist.

8. Verbindersystem nach Anspruch 7, wobei das erste weibliche Verbindungsstück eine zweite Leitung (3) umfasst und das Schraubgewinde einen oder mehrere Vorsprünge (8) auf der Außenseite der genannten zweiten Leitung (3) aufweist, wobei die erste Leitung (1) in die zweite Leitung (3) eingeführt werden kann, um eine Verbindung zwischen den beiden Leitungen herzustellen, wobei die Öffnung(en) (7) in der Barriere (6) am ersten männlichen Verbindungsstück so positioniert ist/sind, dass die/der Vorsprung/Vorsprünge (8) am ersten weiblichen Verbindungsstück passieren kann/können.

9. Verbindersystem nach Anspruch 8, wobei das System mehrere männliche und weibliche Verbindungsstücke aufweist und die Barriere (6) am ersten männlichen Verbindungsstück und der/die Vorsprung/Vorsprünge (8) am ersten weiblichen Verbindungsstück derart sind, dass sie einen Eingriff zwischen allen anderen weiblichen Verbindungsstücken mit dem Schraubgewinde des ersten männlichen Verbindungsstück verhindern.

10. Verbindersystem nach Anspruch 8, wobei das System mehrere männliche und weibliche Verbindungsstücke aufweist und die Barriere (6) am ersten männlichen Verbindungsstück und der/die Vorsprung/Vorsprünge (8) am ersten weiblichen Verbindungsstück derart sind, dass sie einen Eingriff zwischen wenigstens einem der anderen weiblichen Verbindungsstücke und dem Schraubgewinde des ersten männlichen Verbindungsstück zulassen.

11. Epidurale, intrathekale, regionale Anästhesie, Lokalanästhesie oder intravenöse Vorrichtung mit einem System nach einem der Ansprüche 1 bis 10.

## Revendications

1. Système de raccord permettant de raccorder des conduits à des fins d'écoulement fluidique entre ceux-ci, le système comprenant :
a) un premier raccord mâle ; et
b) un premier raccord femelle,
dans lequel les raccords sont en mesure de se mettre mutuellement en prise de manière amovible pour former un raccordement étanche,
dans lequel le premier raccord mâle est adapté à des fins de raccordement sur le premier raccord femelle, mais pas sur un raccord de blocage femelle Luer standard au moyen d'un filetage de vis qui n'est pas en mesure d'entrer en prise avec le filetage de vis (4) du raccord de blocage femelle Luer standard,
**caractérisé en ce que** le premier raccord femelle est adapté à des fins de raccordement sur le premier raccord mâle et un raccord de blocage mâle Luer standard au moyen d'un filetage de vis qui est en mesure d'entrer en prise avec le filetage de vis du premier raccord mâle et un filetage de vis (5) d'un raccord de blocage mâle Luer standard, et
dans lequel le premier raccord mâle comprend une pluralité de filetages de vis, chaque filetage de vis du premier raccord mâle étant en mesure d'entrer en prise avec le filetage de vis d'un raccord femelle différent.

2. Système de raccord selon la revendication 1, dans lequel le premier raccord mâle comprend un filetage de vis et un moyen destiné à empêcher le filetage de vis (4) d'un raccord de blocage femelle Luer standard d'entrer en prise avec ledit filetage de vis.

3. Système de raccord selon la revendication 2, dans lequel le premier raccord femelle comprend un filetage de vis et le premier raccord mâle comprend un moyen permettant audit filetage de vis d'entrer en prise avec le filetage de vis du premier raccord mâle.

4. Système de raccord selon la revendication 2 ou la revendication 3, dans lequel le moyen permettant d'empêcher le filetage de vis (4) d'un raccord de blocage femelle Luer standard d'entrer en prise avec le filetage de vis du premier raccord mâle comprend une barrière (6).

5. Système de raccord selon la revendication 4, dans lequel le premier raccord mâle comprend un premier conduit (1) retenu à l'intérieur d'un manchon (2), le filetage de vis étant positionné sur l'intérieur du manchon (2), la barrière (6) étant raccordée sur le manchon (2) ou une partie de celui-ci, et étant positionnée à l'entrée ou à l'intérieur du manchon (2).

6. Système de raccord selon la revendication 5, dans lequel la barrière (6) est telle qu'au moins une partie du manchon (2) a une section transversale interne suffisamment petite pour empêcher un raccord de blocage femelle Luer standard d'entrer en prise avec le filetage de vis du premier raccord mâle.

7. Système de raccord selon l'une quelconque des revendications 4 à 6, dans lequel le moyen permettant audit filetage de vis du premier raccord femelle d'entrer en prise avec le filetage de vis du premier raccord mâle comprend une(des) ouverture(s) (7) dans la barrière (6).

8. Système de raccord selon la revendication 7, dans lequel le premier raccord femelle comprend un second conduit (3) et le filetage de vis comprend une ou plusieurs partie(s) saillante(s) (8) sur l'extérieur dudit second conduit (3), le premier conduit (1) étant en mesure d'être inséré dans le second conduit (3) pour former un raccordement entre les deux conduits, la(les) ouverture(s) (7) dans la barrière (6) sur le premier raccord mâle étant positionnée(s) de manière à permettre le passage des partie(s) saillante(s) (8) du premier raccord femelle.

9. Système de raccord selon la revendication 8, dans lequel le système comprend une pluralité de raccords mâles et femelles, et la barrière (6) sur le premier raccord mâle et la(les) partie(s) saillante(s) (8) sur le premier raccord femelle sont telles qu'elles empêchent tous les autres raccords femelles d'entrer en prise avec le filetage de vis du premier raccord mâle.

10. Système de raccord selon la revendication 8, dans lequel le système comprend une pluralité de raccords mâles et femelles, et la barrière (6) sur le premier raccord mâle et la(les) partie(s) saillante(s) (8) sur le premier raccord femelle sont telles qu'elles empêchent au moins l'un des autres raccords femelles d'entrer en prise avec le filetage de vis du premier raccord mâle.

11. Appareil épidural, intrathécal, d'anesthésie régionale, d'anesthésie locale, ou intraveineux, comprenant un système selon l'une quelconque des revendications 1 à 10.
